# Europäisches Patentamt
# European Patent Office
# Office européen des brevets

(11) Publication number: **0 161 722**
**B1**

(12) **EUROPEAN PATENT SPECIFICATION**

(45) Date of publication of patent specification: **04.04.90**

(21) Application number: **85200728.5**

(22) Date of filing: **09.05.85**

(51) Int. Cl.⁵: **C 07 D 205/04**

(54) **Preparation of 1-substituted azetidine-3-ol derivatives.**

(30) Priority: **18.05.84 GB 8412814**

(43) Date of publication of application:
**21.11.85 Bulletin 85/47**

(45) Publication of the grant of the patent:
**04.04.90 Bulletin 90/14**

(84) Designated Contracting States:
**AT BE CH DE FR GB IT LI LU NL SE**

(56) References cited:
**EP-A- 029 265**
**EP-A-0 125 714**

**The Journal of Organic Chemistry, vol. 37, no. 24, 1972 Anderson JR. et al. "The Synthesis of Azetidine-3-carboxylic Acid" pages 3953-3955 Tetrahedron Letters, no. 39, 1966, Pergamon Press Ltd. V.R. Gaertner "Cyclization of 1-Alkylamino-3-halo-2-alkanols to 1-Alkyl-3-azetidinols"**
**Chemical Communications, no. 1, January 10, 1968 S.S. Chatterjee et al. "Synthesis of Azetidin-3-ol" page 93**
**E.S. Dehmlow, S.S. Dehmlow, Phase Transfer Catalysis, 2nd. Ed., 1983, Verlag Chemie, p. 1-4**

(73) Proprietor: **SHELL INTERNATIONALE RESEARCH MAATSCHAPPIJ B.V.**
**Carel van Bylandtlaan 30**
**NL-2596 HR Den Haag (NL)**

(72) Inventor: **Wood, Derek Alexander**
**76 Sterling Road**
**Sittingbourne Kent (GB)**
Inventor: **Briner, Paul Howard**
**4 Meadowbank Cottages Painters Forstal**
**Faversham Kent (GB)**

(74) Representative: **Bennett, David Arthur Horder et al**
**4, York Road**
**London SE1 7NA (GB)**

Courier Press, Leamington Spa, England.

## Description

This invention relates to a process for the preparation of 1-substituted azetidin-3-ol derivatives which are intermediates for the preparation of biologically active compounds.

It is known, for example, from J. Org. Chem. *37*, 3953 (1972) that 1-benzhydrylazetidin-3-ol may be prepared by the reaction of benzhydrylamine and epichlorohydrin in methanol. However, the introduction of a benzhydryl group is very inconvenient for an economically practicable synthesis route, since the size of that group greatly increases the bulk of material to be processed, only to be removed once its protective function is no longer required. It would be economically very desirable to use a protective group less bulky than the benzhydryl group, but previous published attempts to react epichlorohydrin with a primary amine such as benzylamine have failed to produce any significant yield of the desired cyclised azetidine product.

Applicants found that the use of an aqueous reaction medium enable useful yields of 1-substituted azetidines to be obtained from an epoxy halide and less bulky amines such as benzylamine, which process forms the subject of their copending European Patent Application No. 84200534.0 (Publication No. 125714A). The experiments described in that patent application demonstrated that no azetidine formation occurs when using organic solvents such as acetonitrile, methanol, butanol or ethanediol as reaction medium, but cyclization to an azetidine could be achieved by the use of an aqueous reaction medium. Gaertner in Tetrahedron Letters, *39*, 1966, p 4691—4694 discloses the cyclization of certain alkylamino chloropropanols to form azetidinols using dimethyl sulphoxide as solvents at comparatively low temperature with long reaction times. European Patent Specification No. 29265A describes the cyclization, in the presence of an amine, of esters of certain halopropane carboxylic acids to form alkylcarboxyazetidines.

Applicants have now surprisingly found that, although previous work with some organic solvents failed to achieve cyclization, replacement of the aqueous reaction medium by triethylamine as solvent not only produces the cyclized azetidine, but gives this product in significantly improved yields. Moreover, triethylamine has been found to be unexpectedly specific in producing this yield increase; using other tertiary alkylamines failed to produce any azetidine product. Also, triethylamine is not only apparently unique in its promotion of the cyclization reaction, it is also commercially very convenient because its boiling point (89°C) is convenient for reflux operation. Further, it has been found that triethylamine effectively removes the hydrogen halide generated during the cyclization reaction precipitating the amine hydrohalide, and moreover this precipitate carries with it any non-cyclized polymer which is sometimes formed as an unwanted by-product in the cyclization reaction.

Accordingly the invention provides a process for the preparation of 1-benzyl azetidin-3-ol comprising cyclizing a solution in triethylamine of N-benzyl-3-amino-1-chloropropan-2-ol at elevated temperature.

The cyclization of the aminoalcohol to the azetidine is carried out at elevated temperature, for example from 50° to 150°C, preferably under reflux at the boiling point of the reaction mixture. It has also been found that the cyclization reaction can be usefully accelerated by the inclusion of a phase transfer catalyst, particularly those containing iodide ions. Suitable phase transfer catalysts include tetraalkyl ammonium halides, especially iodides, such as tetrabutyl ammonium iodide. The amount of catalyst is not critical, the lower limit at which a useful rate increase is attained usually being 0.1 mole %, and the upper limit usually being determined by solubility in the triethylamine reaction medium, generally being about 1.6 mole %.

The starting aminoalcohol is conveniently prepared as described in co-pending European application No. 84200534.0, namely by reacting epichlorhydrin with benzylamine. This reaction can be carried out by mixing the reactants in an organic solvent, for example a hydrocarbon solvent such as cyclohexane. Suitable reaction temperatures are from 10°C to 50°C and suitable reaction times are from 12 to 36 hours. The aminoalcohol may be recovered from the reaction mixture by conventional procedures and, if desired, may be purified, for example by recrystallisation, before cyclization.

As mentioned above, the 1-benzyl azetidin-3-ol is a useful intermediate. Thus, it may be converted by known procedures, for example, via the corresponding 3-cyanoazetidine derivative, to azetidine-3-carboxylic acid derivatives, which exhibit plant growth regulant properties, especially the property of rendering sterile the male parts of plants.

The invention is illustrated in the following Examples.

Example 1
Preparation of 1-benzylazetidin-3-ol

N-benzyl-3-amino-1-chloropropan-2-ol (333 g) was taken up in triethylamine (1665 ml) and tetrabutylammonium iodide (10 g) added. The reaction mixture was stirred under reflux for 13 hours, after which it was cooled, the hydrochloride precipitate filtered off and then washed twice with triethylamine. The combined filtrates were evaporated to yield 252 g of an oil, which was recrystallized from toluene (250 ml) and hexane (50 ml) to yield 180.8 g of white crystals, m.pt. 66—67°C, being a yield (based on aminopropanol) of 66.5%.

Example 2
Use of 1-benzylazetidin-3-ol to prepare azetidin-3-carboxylic acid

(a) 1-Benzylazetidin-3-ol (5.0 g), methane sulphonyl chloride (3.52 g) and triethylamine (6 ml) in dichloromethane (40 ml) were stirred together for 18 hours. The mixture was filtered and the solvent was removed under reduced pressure. The residue was purified by chromatography on silica gel using isopropanol in dichloromethane as eluent to give the mesylate of 1-benzylazetidin-3-ol, yield 4.25 g.

(b) The mesylate of 1-benzylazetidin-3-ol (1.7 g, prepared as in (a)), and sodium cyanide (1.2 g) were stirred together in water (1 ml) and dimethylformamide (20 ml) at 60°C for 16 hours. The solvent was removed under reduced pressure and the residue was purified by chromatography on silica gel using isopropanol in dichloromethane as eluent to give 1-benzyl-3-cyanoazetidine, yield 0.5 g.

(c) 1-Benzyl-3-cyanoazetidine (0.5 g, prepared as in (b)) in saturated barium hydroxide solution (10 ml) was heated under reflux for 30 hours. The reaction mixture was cooled, saturated with gaseous carbon dioxide and filtered. The solvent was removed from the filtrate under reduced pressure to give 1-benzylazetidine-3-carboxylic acid in 80% yield.

(d) 1-Benzylazetidine-3-carboxylic acid (0.5 g), prepared as in (c)) in methanol (15 ml) was hydrogenated in the presence of a 5% palladium on carbon catalyst at room temperature. The catalyst was filtered off and the solvent removed from the filtrate under reduced pressure to give azetidin-3-carboxylic acid in 90% yield.

Example 3

Procedures similar to that of Example 1 were carried out, but omitting the tetrabutylammonium iodide, and also replacing that phase transfer catalyst by the bromide and by sodium iodide. In all cases a comparable yield of product could be achieved (around 65%), though longer reaction times were required.

Comparative experiments

The procedure of Example 1 was repeated, except that in step (b) the triethylamine was replaced with tributylamine, tripropylamine, diisopropylethylamine, pyrrolidine, pyridine or with 2,6-lutidine. In no case did the reaction produce any significant yield of azetidinol, thus clearly demonstrating the unexpected specificity of triethylamine in promoting a high yield of that product.

**Claims**

1. A process for preparation of 1-benzyl azetidin-3-ol by cyclizing a 3-amino-1-halopropane derivative characterised in that the azetidinol is prepared by cyclizing a solution in triethylamine of the propane derivative N-benzyl-3-amino-1-chloro-propan-2-ol at elevated temperature.

2. A process as claimed in claim 1, wherein the cyclization is carried out in the presence of a phase-transfer catalyst.

3. A process as claimed in claim 2, wherein the phase-transfer catalyst is a tetraalkylammonium halide.

4. A process as claimed in any one of the preceding claims wherein the cyclization is effected by heating at a temperature from 50° to 150°C.

5. A process as claimed in claim 4, wherein the cyclization is carried out under reflux.

**Patentansprüche**

1. Verfahren zur Herstellung von 1-Benzylazetidin-3-ol durch Zyklisieren eines 3-Amino-1-halogenpropan-Derivats, dadurch gekennzeichnet, daß das Azetidinol durch Zyklisieren einer Lösung des Propanderivats N-Benzyl-3-amino-1-chlorpropan-2-ol in Triethylamin bei erhöhter Temperatur hergestellt wird.

2. Verfahren nach Anspruch 1, worin die Zyklisierung in Anwesenheit eines Phasentransfer-Katalysators ausgeführt wird.

3. Verfahren nach Anspruch 2, worin der Phasentransfer-Katalysator ein Tetraalkylammoniumhalogenid ist.

4. Verfahren nach einem der vorstehenden Ansprüche, worin die Zyklisierung durch Erhitzen auf eine Temperatur von 50 bis 150°C bewirkt wird.

5. Verfahren nach Anspruch 4, worin die Zyklisierung unter Rückflußsieden ausgeführt wird.

**Revendications**

1. Un procédé pour la préparation du benzyl-1 azétidinol-3 par cyclisation d'un dérivé amino-3 halo-1 propane, caractérisé en ce que l'azétidinol préparé par cyclisation d'une solution dans la triéthylamine du dérivé du propane, le N-benzyl-3-amino-1-chloro-propanol-2 à température élevée.

2. Un procédé tel que revendiqué dans la revendication 1, dans lequel la cyclisation est réalisée en présence d'un catalyseur de transfert de phase.

3. Un procédé tel que revendiqué dans la revendication 2, dans lequel le catalyseur de transfert de phase est un halogénure tétraalkylammonium.

4. Un procédé tel que revendiqué dans l'une quelconque des revendications précédentes, dans lequel la cyclisation est effectuée par chauffage à une température de 50° à 150°C.

5. Un procédé tel que revendiqué dans la revendication 4, dans lequel la cyclisation est réalisée au reflux.